# EUROPEAN PATENT SPECIFICATION

(11) **EP 4 056 116 B1**
(45) Date of publication and mention of the grant of the patent: **17.09.2025**
(21) Application number: 20885459.6
(22) Date of filing: 20.10.2020
(51) Int. Cl.: A61B 5/1468, A61B 5/145, A61B 5/157, A61B 5/00, G01N 33/52, G01N 33/487, H04M 1/725, A61B 10/00, A61B 5/103, A61B 5/1455, A61B 5/1495, A61B 5/15, G01N 21/78

(54) **LIGHT CONTROL APPARATUS AND METHOD FOR COLORIMETRIC METHOD**
LICHTSTEUERVORRICHTUNG UND -VERFAHREN FÜR EIN KOLORIMETRISCHES VERFAHREN
APPAREIL ET PROCÉDÉ DE COMMANDE DE LUMIÈRE POUR PROCÉDÉ COLORIMÉTRIQUE

(30) Priority: 06.11.2019 KR 20190140887
(43) Date of publication of application: 14.09.2022
(73) Proprietor: 1Drop Inc., Gyeonggi-do 13217 (KR)
(72) Inventor: BAEK, So Hyeon, Seoul 02811 (KR); CHOI, Hoon Ki, Gwangju-si Gyeonggi-do 12771 (KR); CHOI, Ko Bong, Yongin-si Gyeonggi-do 16872 (KR); RHEE, Joo Won, Seongnam-si Gyeonggi-do 13562 (KR)
(74) Representative: advotec.
(86) International application number: PCT/KR2020/014320
(87) International publication number: WO 2021/091121

(56) References cited:
- EP-A2- 2 702 766
- CN-A- 109 770 852
- JP-A- 2012 179 212
- KR-A- 20090 036 996
- KR-A- 20100 008 073
- KR-A- 20150 006 114
- KR-A- 20170 133 238
- KR-B1- 101 062 356
- KR-B1- 102 136 194
- US-A1- 2009 002 544
- US-A1- 2017 234 858
- US-A1- 2017 343 480
- US-A1- 2019 219 589

## Description

### [Technical Field]

The present invention relates to a colorimetric method through camera capturing, and more specifically, to an apparatus and method capable of performing a more accurate colorimetric method measurement through light control.

### [Background Art]

This application claims priority based on Patent Application No. 10-2019-0140887 applied in the Republic of Korea on October 24, 2019.

A measurement method using a test strip, that is, a sensor strip (biosensor strip), is used to detect a specific level through a sample such as blood, urine, or saliva. For example, when blood glucose is measured, the sensor strip is inserted into an inlet of a blood glucose monitoring device, blood is drawn from a fingertip, and a small amount of blood collected is injected into the sensor strip. The sensor strip has a membrane to which a reagent is applied. The injected blood reacts with the reagent, and the blood glucose can be numerically calculated through a measurement device according to a degree of the reaction.

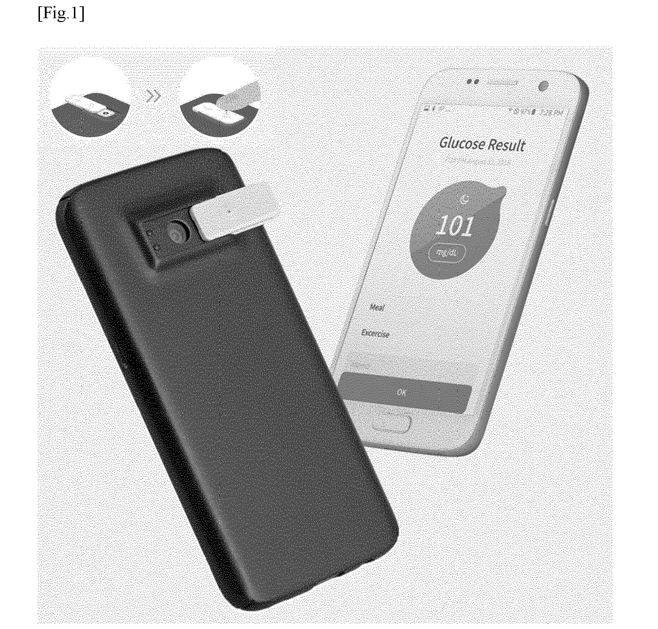

FIG. 1 is an exemplary view of a measurement device capable of executing a colorimetric method using a camera of a smartphone.

Referring to FIG. 1, it can be seen that a sensor strip is mounted in front of the camera of the smartphone. A reagent applied to the sensor strip causes a color reaction in proportion to a blood glucose level, and the measurement device is an apparatus which captures the color reaction using the camera and analyzes. The reaction of the reagent is described in more detail as shown in the following Reaction Scheme 1.

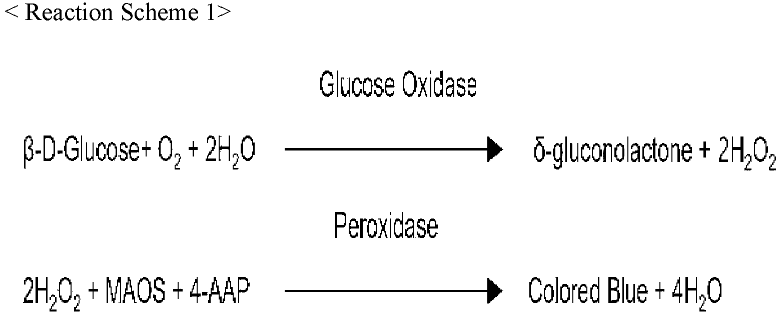

That is, a chromogenic compound (MAOS) appears blue depending on the blood glucose level. A bio-measurement device using the camera of the smart phone can measure blood glucose in a way that measures an amount of blue in an image captured by the camera and converts the amount of blue to the blood glucose level. This method of measuring a level of a component in the blood (for example, blood glucose) through color is referred to as a colorimetric method.

US 2017/234858 A1 discloses a reflectance based, colorimetric test strip reader for use with a mobile device having a jack plug receiving socket.

From US 2017/343480 A1 a method for measuring blood glucose levels by a portable terminal using a strip module is knwon. The strip module includes a dye pad having a color that changes in response to a sample applied to the dye pad. The strip module also includes a transparent strip having a first side and a second side.

KR 2017 0133238 A teaches a strip module and a method for a portable terminal to acquire information on a sample using the strip module. The strip module includes a dye pad in which the color changes in response to an injected sample and a transparent strip in which the dye pad is mounted on one surface. Light supplied from a light source of the portable terminal through the one surface is reflected to be transferred to the dye pad.

### [Disclosure]

### [Technical Problem]

The present specification is directed to providing an apparatus and method for controlling the brightness of light to calculate a more accurate level compared to a case in which a level of a component in blood is measured through a change in color of a reaction reagent.

The present specification is not limited to the above-mentioned problems, and other problems which are not mentioned will be clearly understood by those skilled in the art from the following disclosure.

### [Technical Solution]

The invention is in accordance with the appended claims.

### [Description of Drawings]

FIG. 1 is an exemplary view of a measurement device capable of executing a colorimetric method using a camera of a smartphone.
FIG. 2 is a graph illustrating changes in R values for different blood glucose levels.
FIG. 3 is a block diagram schematically illustrating a configuration of a light control apparatus according to the present specification.
FIG. 4 is an exemplary view illustrating a change in R value measured when the brightness is changed according to the present specification.
FIG. 5 is an exemplary view illustrating a difference in measured values according to blood glucose.
FIG. 6 is a flow chart of a light control method for a colorimetric method according to the present specification.
FIG. 7 is a block diagram schematically illustrating a configuration of a light control apparatus according to another embodiment of the present specification.
FIG. 8 is a flow chart of a light control method for a colorimetric method according to another embodiment of the present specification.
FIG. 9 is a block diagram schematically illustrating a configuration of a light control apparatus according to still another embodiment of the present specification.
FIG. 10 is a flow chart of a light control method for a colorimetric method according to still another embodiment of the present specification.

### [Modes of the Invention]

Advantages and features of the present invention disclosed in the present specification, and a method of achieving them, will become apparent with reference to embodiments which are described in detail in conjunction with the accompanying drawings. However, the present specification is not limited to the embodiments to be described below and may be implemented in different forms, but the embodiments are only provided to completely disclose the present invention and completely convey the scope of the present invention to those skilled in the art, and the present invention is only defined by the disclosed claims. Hereinafter, the embodiments of the present invention will be described in detail with reference to the accompanying drawings

A level of a specific component in a sample, specifically, a level of a specific component in blood through a colorimetric method has a close relationship with a value of a color which appears due to a chromogenic compound in image data (hereinafter referred to as 'a chromogenic value'). In a digital image, various colors are expressed using red (R), green (G), and blue (B). However, each of the RGB values has a range of 0 to 255. Accordingly, even when a process in which the color changes according to a reaction process between a sample and a reagent is captured, a measurement range in which each RGB value is maximally expressed in the digital image is in a range between 0 and 255.

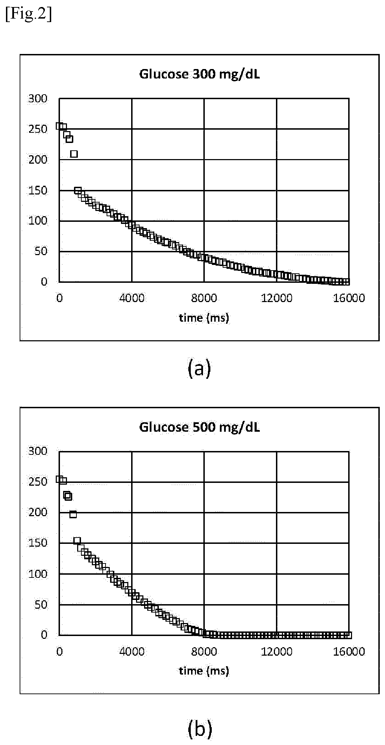

FIG. 2 is a graph illustrating changes in R values for different blood glucose levels.

FIG. 2A illustrates a case in which blood glucose is 300 mg/dL, and FIG. 2B illustrates a case in which blood glucose is 500 mg/dL. Since both cases start at an R value of 255 and the R value becomes 0 after 15 seconds, the case in which the blood glucose is 300 mg/dL and the case in which the blood glucose is 500 mg/dL cannot be distinguished. An RGB color coordinate system, a CIE 1931 color coordinate system, XYZ, YIQ, YUV, or the like may be used as the coordinate system. However, the above-described problem is a phenomenon which may appear regardless of the color coordinate system. In the present specification, each color will be described with respect to the RGB coordinate system in which values range from 0 to 255. However, the color coordinate system to be described in the present specification is only an example, and the present specification is not limited thereto.

Further, there are various types of chromogenic compounds which react with a glucose component in blood and may have different chromogenic values. For example, when a combination of MAOS and 4-APP comes into contact with blood, a chromogenic value has a wavelength of 630 nm (blue). As another example, when a combination of TOOS and 4-APP comes into contact with blood, a chromogenic value has a wavelength of 555 nm (purple). As still another example, when a combination of DAOS and 4-APP comes into contact with blood, a chromogenic value has a wavelength of 593 nm (sky blue). However, in the present specification, the color will be described based on the change in R value.

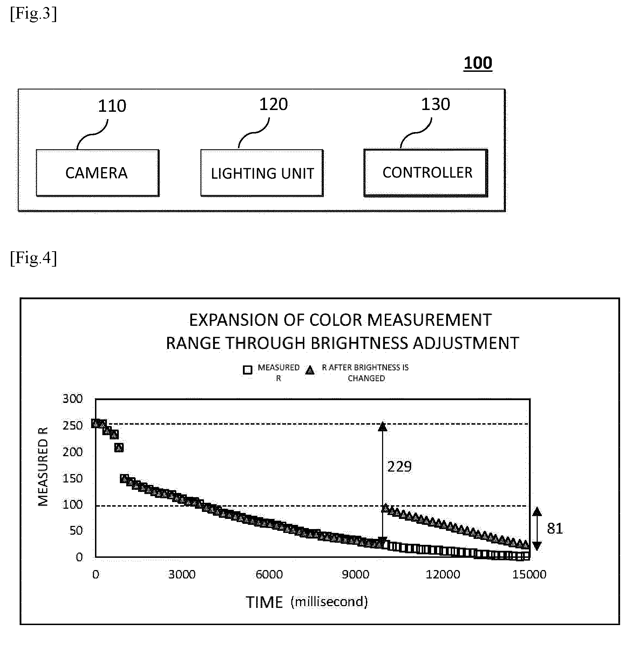

FIG. 3 is a block diagram schematically illustrating a configuration of a light control apparatus according to one embodiment of the present specification.

Referring to FIG. 3, a light control apparatus 100 according to one embodiment of the present specification may include a camera 110, a lighting unit 120, and a controller 130.

The camera 110 may output an image signal. Meanwhile, the light control apparatus 100 according to the present specification may be one element of a blood component measurement device or a mobile communication terminal (for example, a smart phone). In this case, a coupling part (not shown) through which a sensor strip is coupled to a housing surrounding the outside of the blood component measurement device or the mobile communication terminal may be formed. The camera 110 is used for capturing a reaction of blood and a reagent in the sensor strip, and the coupling part may be formed at a position where the camera 110 may capture the sensor strip when the sensor strip is coupled (See FIG. 1).

The lighting unit 120 may emit light according to a control signal. The lighting unit 120 may include various types of light sources (for example, light emitting diodes (LEDs)) capable of outputting light. The control signal is a signal capable of controlling a brightness of light to be emitted as well as controlling an activation state in which the lighting unit 120 emits light and an inactivation state in which the lighting unit 120 does not emit light. The lighting unit 120 may be installed at a position adjacent to the camera 110 to illuminate a reaction portion of the blood and the reagent in the sensor strip.

The controller 130 may store a reaction image of the reagent and the sample output by the camera 110 as image data. Further, the controller 130 may output a first control signal so that the lighting unit 120 outputs light with a first brightness, and may output a second control signal so that the lighting unit 120 outputs light with a second brightness having a different brightness from the first brightness during a reaction of the reagent and the sample. In this case, the second brightness may be brighter than the first brightness.

FIG. 4 is an exemplary view illustrating a change in R value measured when the brightness is changed according to the present specification.

Referring to FIG. 4, a graph indicated by squares and a graph indicated by triangles may be identified. The graphs shown in FIG. 3 are graphs in which the R values are measured over time while reacting blood having an arbitrary blood glucose level with a reagent. First, the graph indicated by the squares illustrates a case in which the brightness of light is constant at the first brightness during the reaction. Accordingly, in the graph indicated by the squares, the R value gradually decreases from 255 to 0. On the other hand, the graph indicated by the triangles illustrates a case in which the brightness of light is changed from the first brightness to the second brightness in the middle of the reaction (10 seconds after the reaction). Since the second brightness is brighter than the first brightness, it can be seen that the R value is changed from 21 to 100. An image sensor (charge coupled device, CCD) of the camera 110 is a device which converts the intensity of light to an electrical signal. Accordingly, even in the case of a target, the intensity of light measured by the image sensor may be different according to the brightness of external light.

After the brightness of the light is changed to the second brightness, the measured R value is from 100 to 19, and thus a difference is 81. In the graph in which the brightness of the light is constant at the first brightness, the R value in the same section is from 21 to 0, and thus a difference is 21. Accordingly, when two graphs are compared in the same section, it can be seen that a color measurement range is expanded from 21 to 81. When comparing the entire section, the conventional measurement range is 255 to 0, but the measurement range acquired by changing the brightness of the light is 255 to -60, and thus there is an effect in that the measurement range is expanded by approximately 123%.

According to the present specification, the controller 130 changes the first control signal to the second control signal when the image signal output by the camera 110 is smaller than or equal to a preset reference signal value. The reference signal value may be variously set through various methods such as an experiment or the like.

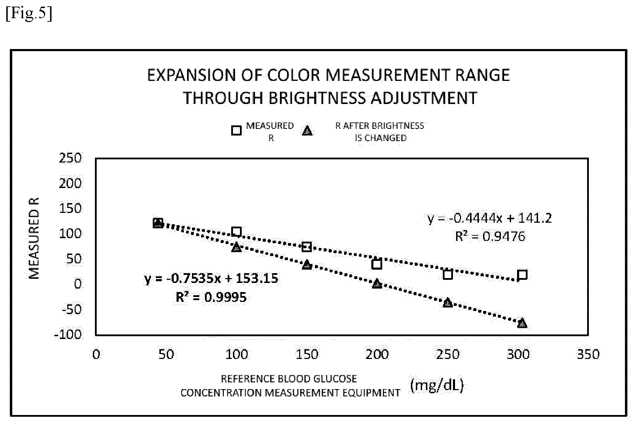

FIG. 5 is an exemplary view illustrating a difference in measured values according to blood glucose.

Referring to FIG. 5, an X-axis indicates a blood glucose level, and a Y-axis indicates the measured R value. A graph indicated by the squares illustrates a case which is constantly measured at the first brightness. A graph indicated by triangles illustrates a case measured after the brightness is changed from the first brightness to the second brightness . As shown in FIG. 5, since the measurement range when the brightness is changed is wider, a difference in the R value according to a blood glucose difference may be more clearly distinguished. For example, the R values measured at a blood glucose level of 250 mg/dL and a blood glucose level of 300 mg/dL will be compared. When the brightness does not change, since the R value actually measured at the blood glucose level of 250 mg/dL and the R value actually measured at the blood glucose level of 300 mg/dL are almost similar, a difference between the two blood glucose levels may not be distinguished using the measured values. On the other hand, when the brightness is changed, since the R value actually measured at the blood glucose level of 250 mg/dL and the R value actually measured at the blood glucose level of 300 mg/dL are clearly different, the difference between the two glucose levels may be distinguished. It is self-evident that the wider the measurement range, the better blood glucose discrimination, and accordingly, repeatability of the measurement value may be improved. It is clear that the improvement of repeatability is a factor directly related to the improvement of accuracy and reliability of the measurement device.

Hereinafter, a light control method for a colorimetric method according to one embodiment of the present specification will be described. However, in a description of the light control method according to the present specification, a repetitive description of each configuration of the light control apparatus will be omitted.

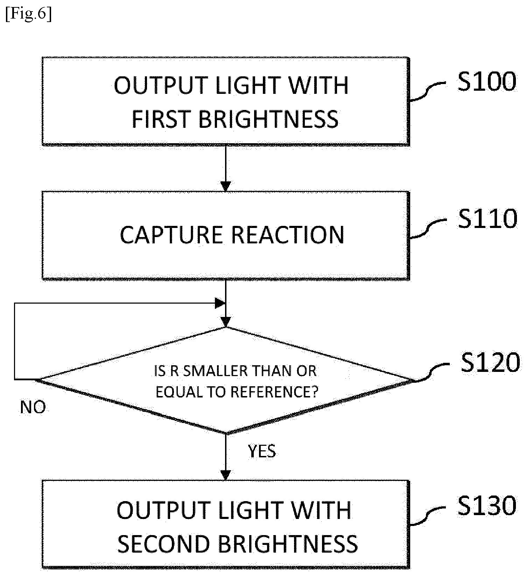

FIG. 6 is a flow chart of a light control method for a colorimetric method according to one embodiment reflecting the invention as claimed.

Referring to FIG. 6, first, in operation S100, the controller 130 outputs the first control signal so that the lighting unit 120 outputs light with the first brightness. In subsequent operation S110, the controller 130 controls the camera 110 to capture the reaction image between the reagent and the sample. In this case, the controller 130 monitors whether the image signal output by the camera 110 is smaller than or equal to the preset reference signal value in operation S120. In subsequent operation S130, the controller 130 outputs the second control signal so that the lighting unit 120 outputs light with the second brightness having a brightness different from the first brightness. In this case, the second brightness may be brighter than the first brightness.

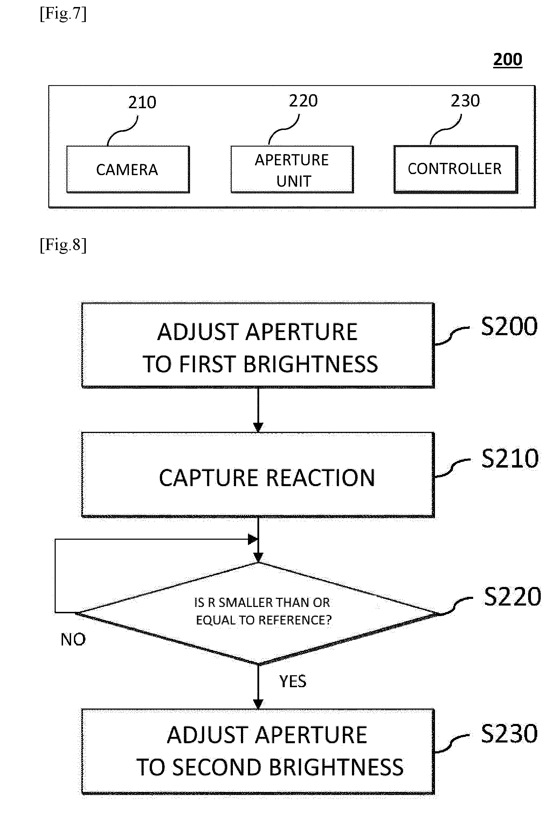

FIG. 7 is a block diagram schematically illustrating a configuration of a light control apparatus according to another embodiment of the present specification

Referring to FIG. 7, a light control apparatus 200 according to another embodiment of the present specification may include a camera 210, an aperture unit 220, and a controller 230.

The camera 210 is the same camera as the camera 110 included in the light control apparatus 100 according to one embodiment of the present specification, and thus a repetitive description thereof will be omitted.

The aperture unit 220 may adjust an amount of light which enters the camera 210.

The controller 230 is similar to the controller 130 included in the light control apparatus 100 according to one embodiment of the present specification, but controls the brightness by controlling the aperture unit 220 instead of the lighting unit 120. Specifically, the controller 230 may output a first control signal to the aperture unit 220 so that the amount of light which enters the camera 210 may become a first brightness, and, and may output a second control signal to the aperture unit 220 so that the amount of light which enters the camera 210 may become a second brightness having a brightness different from the first brightness during a reaction of a reagent and a sample. When the second brightness is brighter than the first brightness, a degree of opening of the aperture according to the second control signal may be greater than a degree of opening of the aperture according to the first control signal.

In another embodiment of the present specification, since an effect according to a change from the first control signal to the second control signal of the controller 230 is similar to a case described with reference to FIGS. 4 and 5, a repetitive description will be omitted.

FIG. 8 is a flow chart of a light control method for a colorimetric method according to another embodiment of the present specification.

Referring to FIG. 8, first, in operation S200, the controller 230 may output the first control signal to the aperture unit 220 so that the amount of light which enters the camera 210 may become the first brightness. In subsequent operation S210, the controller 230 may control the camera 210 to capture the reaction image between the reagent and the sample. In this case, the controller 230 may monitor whether the image signal output by the camera 210 is smaller than or equal to a preset reference signal value in operation S220. In subsequent operation S230, the controller 230 may output the second control signal to the aperture unit 220 so that the amount of light which enters the camera 210 may become the second brightness having a brightness different from the first brightness. In this case, the second brightness may be brighter than the first brightness.

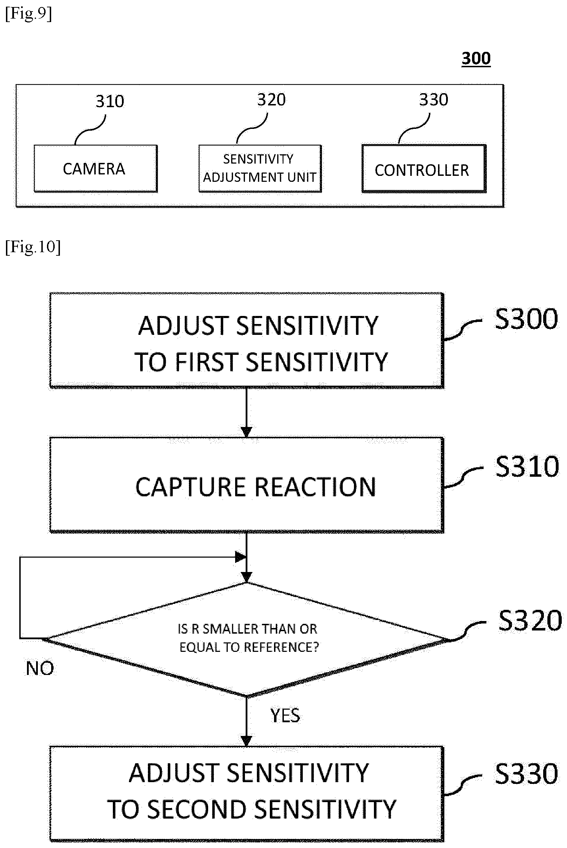

FIG. 9 is a block diagram schematically illustrating a configuration of a light control apparatus according to still another embodiment of the present specification.

Referring to FIG. 9, a light control apparatus 300 according to still another embodiment of the present specification may include a camera 310, a sensitivity adjustment unit 320, and a controller 330.

The camera 310 is the same camera as the camera 110 included in the light control apparatus 100 according to one embodiment of the present specification, and thus a repetitive description thereof will be omitted.

The sensitivity adjustment unit 320 may adjust the sensitivity of a plurality of pixel arrays included in the camera 210. 'Sensitivity' is a physical value which indicates a degree to which a camera sensor, that is, a plurality of pixel arrays, responds to light, and may be expressed as International Organization for Standardization (ISO).

The controller 330 is similar to the controller 130 included in the light control apparatus 100 according to one embodiment of the present specification, but controls the brightness by controlling the sensitivity adjustment unit 320 instead of the lighting unit 120. Specifically, the controller 330 may output a first control signal to the sensitivity adjustment unit 320 so that the sensitivity of light sensed by the plurality of pixel arrays may become a first sensitivity, and, and may output a second control signal to the sensitivity adjustment unit 320 so that the sensitivity of light sensed by the plurality of pixel arrays may become a second sensitivity having a sensitivity different from the first sensitivity during a reaction of a reagent and a sample. The sensitivity according to the second control signal may be greater than the sensitivity according to the first control signal.

In still another embodiment of the present specification, since an effect according to a change from the first control signal to the second control signal of the controller 330 is similar to a case described with reference to FIGS. 4 and 5, a repetitive description will be omitted.

FIG. 10 is a flow chart of a light control method for a colorimetric method according to still another embodiment of the present specification.

Referring to FIG. 10, first, in operation S300, the controller 330 may output the first control signal to the sensitivity adjustment unit 320 so that the sensitivity of light sensed by the plurality of pixel arrays may become the first sensitivity. In subsequent operation S310, the controller 330 may control the camera 310 to capture the reaction image between the reagent and the sample. In this case, the controller 330 may monitor whether the image signal output by the camera 210 is smaller than or equal to a preset reference signal value in operation S320. In subsequent operation S330, the controller 230 may output the second control signal to the sensitivity adjustment unit 320 so that the sensitivity of light sensed by the plurality of pixel arrays may become the second sensitivity having a sensitivity different from the first sensitivity. In this case, the second sensitivity may be brighter than the first sensitivity.

The controller 130, 230, or 330 may include a processor, an application-specific integrated circuit (ASIC), other chipsets, a logic circuit, a register, a communication modem, a data processing unit, and the like known in the art of the present invention to perform calculation and various control logics. Further, when the above-described control logics are implemented in software, the controller 130 may be implemented as a set of program modules. In this case, the program modules may be stored in a memory and executed by the processor.

The computer program may include code coded in a computer language such as C/C++, C#, JAVA, Python, and machine language which may be read by a processor (CPU) of the computer through a device interface of the computer so that the computer can read the program and execute the methods realized as a program. Such code may include functional code related to a function which defines functions necessary for executing the methods, and the like, and may include control code related to an execution procedure necessary for the processor of the computer to execute the functions according to a predetermined procedure. Further, the code may further include code related to memory reference for a position (address number) of an internal memory or external memory of the computer where additional information or media necessary for the processor of the computer to execute the above-described functions should be referenced. In addition, when the processor of the computer needs to communicate with another computer, a server, or the like which is remotely located in order to execute the above-described functions, the code may further include communication-related code for how to communicate with another computer, the server, or the like which is remotely located, using the communication module of the computer and for what information or media to transmit and receive during communication, and the like.

The stored medium is not a medium which stores data for a short moment, such as a register, a cache, a memory, or the like, but a medium which semi-permanently stores data and is readable by a device. Specifically, examples of the stored medium may include, a read only memory (ROM), a random access memory (RAM), a CD-ROM, a magnetic tape, a floppy disk, an optical data storage device, or the like, but the present invention is not limited thereto. That is, the program may be stored in various recording media on various servers to which the computer may be linked or in various recording media on the user's computer. Further, the medium may be distributed in a computer system connected through a network, and computer-readable code may be stored in a distributed manner.

In the above, although embodiments of the present specification have been described with reference to the accompanying drawings, those skilled in the art may understand that the present invention may be embodied in other specific forms without changing the essential features thereof. Accordingly, it should be understood that the above-described embodiments are exemplary in all respects and not restrictive.

### [Reference numerals]

100, 200, 300: light control apparatus
110, 210, 310: camera
120: lighting unit
130, 230, 330: controller
220: aperture unit
320: sensitivity adjustment unit

## Claims

1. A light control apparatus for a colorimetric method, comprising:
a camera (110) configured to capture a reaction image of a reagent and a sample, and to output an image signal;
a lighting unit (120) configured to emit light to a reaction portion of the reagent and the sample according to a control signal; and
a controller configured to store a reaction image of a reagent and a sample output by the camera (110) as image data, output a first control signal so that the lighting unit (120) outputs light with a first brightness, **characterised in that** the controller is further configured to change the first control signal to a second control signal and to output the second control signal when the image signal output by the camera (110) is smaller than or equal to a preset reference signal value and is configured to output the second control signal so that the lighting unit (120) outputs light with a second brightness brighter than the first brightness during a reaction of the reagent and the sample.

2. A light control method for a colorimetric method using a device including a camera (110) configured to capture a reaction image of a reagent and a sample, and to output an image signal, a lighting unit (120) configured to emit light to a reaction portion of the reagent and the sample according to a control signal, and a controller (130), the light control method comprising:
(a) outputting, by the controller (130), a first control signal to the lighting unit (120) to output light with a first brightness;
(b) controlling, by the controller (130), the camera (110) to capture a reaction image of a reagent and a sample; and **characterised by** :
(c) outputting, by the controller (130), a second control signal to the lighting unit (120) to output light with a second brightness brighter than the first brightness when the image signal output by the camera (110) is smaller than or equal to a preset reference signal value.

## Patentansprüche

1. Lichtsteuerungsgerät für ein kolorimetrisches Verfahren, Folgendes umfassend:
eine Kamera (110), die dazu konfiguriert ist, ein Reaktionsbild eines Reagens und einer Probe zu erfassen und ein Bildsignal auszugeben;
eine Beleuchtungseinheit (120), die dazu konfiguriert ist, in Abhängigkeit von einem Steuersignal Licht an einen Reaktionsabschnitt des Reagens und der Probe zu emittieren; und
eine Steuerung, die dazu konfiguriert ist, ein Reaktionsbild eines Reagens und einer Probe, die von der Kamera (110) als Bilddaten ausgegeben werden, zu speichern, und ein erste Steuersignal auszugeben, so dass die Beleuchtungseinheit (120) Licht mit einer ersten Helligkeit ausgibt, **dadurch gekennzeichnet, dass** die Steuerung des Weiteren dazu konfiguriert ist, das erste Steuersignal in ein zweites Steuersignal zu ändern und das zweite Steuersignal auszugeben, wenn das von der Kamera (110) ausgegebene Bildsignal kleiner als oder gleich einem voreingestellten Referenzsignalwert ist,
und dazu konfiguriert ist, das zweite Steuersignal auszugeben, so dass die Beleuchtungseinheit (120) Licht mit einer zweiten Helligkeit ausgibt, die während einer Reaktion des Reagens und der Probe heller als die erste Helligkeit ist.

2. Lichtsteuerungsverfahren für ein kolorimetrisches Verfahren, das eine Vorrichtung verwendet, die eine Kamera (110), die dazu konfiguriert ist, ein Reaktionsbild eines Reagens und einer Probe zu erfassen und ein Bildsignal auszugeben, eine Beleuchtungseinheit (120), die dazu konfiguriert ist, in Abhängigkeit von einem Steuersignal Licht an einen Reaktionsabschnitt des Reagens und der Probe zu emittieren, und eine Steuerung (130) umfasst, wobei das Lichtsteuerungsverfahren folgende Schritte umfasst:
(a) Ausgeben eines ersten Steuersignals an die Beleuchtungseinheit (120) durch die Steuerung (130), um Licht mit einer ersten Helligkeit auszugeben;
(b) Steuern der Kamera (110) durch die Steuerung (130), um ein Reaktionsbild eines Reagens und einer Probe zu erfassen, und **gekennzeichnet durch**
(c) das Ausgeben eines zweiten Steuersignals an die Beleuchtungseinheit (120) durch die Steuerung (130), um Licht mit einer zweiten Helligkeit auszugeben, die heller als die erste Helligkeit ist, wenn das von der Kamera (110) ausgegebene Bildsignal kleiner als oder gleich einem voreingestellten Referenzsignalwert ist.

## Revendications

1. Appareil de contrôle de lumière pour un procédé colorimétrique, ledit appareil de contrôle de lumière comprenant :
une caméra (110) configurée pour capturer une image de réaction d'un réactif et d'un échantillon, et pour émettre un signal d'image ;
une unité d'éclairage (120) configurée pour émettre de la lumière vers une partie de réaction du réactif et de l'échantillon en fonction d'un signal de contrôle ; et
un dispositif de contrôle configuré pour stocker une image de réaction d'un réactif et d'un échantillon émises par la caméra (110) comme données d'image et pour émettre un premier signal de contrôle de sorte que l'unité d'éclairage (120) émette de la lumière avec une première luminosité, **caractérisé en ce que** le dispositif de contrôle est en outre configuré pour changer le premier signal de contrôle en un deuxième signal de contrôle et pour émettre le deuxième signal de contrôle lorsque le signal d'image émis par la caméra (110) est inférieur ou égal à une valeur préréglée de signal de référence et est configuré pour émettre le deuxième signal de contrôle de sorte que l'unité d'éclairage (120) émette de la lumière avec une deuxième luminosité qui est plus lumineuse pendant une réaction du réactif et de l'échantillon que la première luminosité.

2. Procédé de contrôle de lumière pour un procédé colorimétrique utilisant un dispositif comprenant une caméra (110) configurée pour capturer une image de réaction d'un réactif et d'un échantillon, et pour émettre un signal d'image, une unité d'éclairage (120) configurée pour émettre de la lumière vers une partie de réaction du réactif et de l'échantillon en fonction d'un signal de contrôle, et un dispositif de contrôle (130), le procédé de contrôle de lumière comprenant les étapes suivantes :
(a) émettre un premier signal de contrôle vers l'unité d'éclairage (120) par le dispositif de contrôle (130) pour émettre de la lumière avec une première luminosité ;
(b) contrôler la caméra (110) par le dispositif de contrôle (130) pour capturer une image de réaction d'un réactif et d'un échantillon ; et **caractérisé par**
(c) l'étape consistant à émettre un deuxième signal de contrôle vers l'unité d'éclairage (120) par le dispositif de contrôle (130) pour émettre de la lumière avec une deuxième luminosité qui est plus lumineuse que la première luminosité lorsque le signal d'image émis par la caméra (110) est inférieur ou égal à une valeur préréglée de signal de référence.
